(19) Europäisches Patentamt
European Patent Office
Office européen des brevets

(11) Numéro de publication : 0 325 534 B1

(12) FASCICULE DE BREVET EUROPEEN

(45) Date de publication du fascicule du brevet : 02.05.91 Bulletin 91/18

(51) Int. Cl.[5] : C12P 13/00, // C12N1/10, (C12P13/00, C12R1:645)

(21) Numéro de dépôt : 89400150.2

(22) Date de dépôt : 19.01.89

# (54) Procédé de synthèse de mononitrates de glycéryle par bioconversion de la nitroglycérine.

(30) Priorité : 20.01.88 FR 8800603

(43) Date de publication de la demande : 26.07.89 Bulletin 89/30

(45) Mention de la délivrance du brevet : 02.05.91 Bulletin 91/18

(84) Etats contractants désignés : AT BE CH DE ES FR GB GR IT LI LU NL SE

(56) Documents cités :
APPLIED AND ENVIRONMENTAL MICROBIOLOGY, vol. 36, no. 5, 1978, pages 693-699, American Society for Microbiology, US; T.M. WENDT et al.: "Microbial degradation of glycerol nitrates"
APPLIED AND ENVIRONMENTAL MICROBIOLOGY, vol. 43, no. 1, janvier 1982, pages 144-150; D.L. KAPLAN et al.: "Biodegradation of glycidol and glycidyl nitrate"

(73) Titulaire : SOCIETE NATIONALE DES POUDRES ET EXPLOSIFS
12, quai Henri IV
F-75181 Paris Cédex 04 (FR)

(72) Inventeur : Ducrocq, Claire
25 Av. des Cottages
F-91400 Orsay (FR)
Inventeur : Lecolier, Serge
3, Allée des Cartelines
F-91510 Janville sur Juine (FR)
Inventeur : Lenfant, Maryse
18, Rue des Châtaigniers
F-91190 Gif sur Yvette (FR)
Inventeur : Servy, Claudine
148, Rue de Paris
F-91120 Palaiseau (FR)
Inventeur : Wimmer, Eric
30, Allée A. Levèque
F-24100 Bergerac (FR)

(74) Mandataire : Pech, Bernard et al
SNPE - Service Propriété Industrielle 12, Quai Henri IV
F-75181 Paris Cédex 04 (FR)

EP 0 325 534 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un procédé de synthèse des mononitrates de glycéryle par bioconversion de la nitroglycérine.

Plus précisément, l'invention concerne un procédé de bioconversion de la nitroglycérine (trinitrate-1, 2, 3 de glycéryle ou TNG) en un mononitrate de glycéryle ou un mélange de mononitrates de glycéryle.

On distingue deux mononitrates de glycéryle, le mononitrate-1 de glycéryle (MNG-1) de formule (1) :

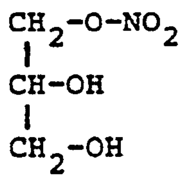

(1)

et le mononitrate-2 de glycéryle (MNG-2) de formule (2) :

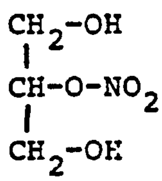

(2)

Ces 2 mononitrates de glycéryle ont été proposés à titre de médicaments dans le traitement de l'angine de poitrine et de l'hypertension pulmonaire, notamment dans les brevets DE 3 514 888 et DE 3 305 690, en particulier sous forme de pastilles sublinguales.

Le développement de ces produits a néanmoins été limité par le fait que le mononitrate-2 n'a, à notre connaissance, jamais pu être préparé avec de bons rendements, mais est seulement récupéré en faible quantité lors de la synthèse du dinitrate.

Quant au mononitrate-1, bien que de nombreuses voies de synthèse chimique soient proposées, voir notamment Rood's Chemistry of Carbon Compounds, seconde édition p 17, aucune d'entre elles n'est satisfaisante.

Lors d'études pharmaco-cinétiques sur la dégradation in vivo de la nitroglycérine, certains auteurs, notamment P. Needleman et F.E. Hunter (Mol. Pharmacol. 1, 77-86 (1965)), mentionnent la formation de traces de mononitrates.

Par ailleurs, T.M. Wendt, J.H, Cornell et A.M. Kaplan, dans Applied and Environmental Microbiology, 36, 5, 693-699 (1978) décrivent la formation à titre transitoire de di-et de mononitrates lors de la transformation de la nitroglycérine en glycérol par fermentation bactérienne.

Ces études antérieures indiquent clairement que les mononitrates ne pouvaient pas être obtenus avec de bons rendements et à fortiori qu'il n'était pas possible d'obtenir sélectivement un des mononitrates vis à vis de l'autre puisque, ou bien le produit était obtenu à l'état de trace, ou bien le produit final de fermentation était le glycérol.

La demanderesse a découvert que, de façon inattendue, certains microorganismes permettaient d'obtenir les mononitrates à partir de la nitroglycérine, et même sélectivement l'un des mononitrates vis à vis de l'autre, avec des rendements élevés et un degré élevé de pureté, en une seule étape et dans un milieu réactionnel exempt de toute espèce minérale cationique ou anionique, sans traitements complexes de purification.

Le procédé selon l'invention est un procédé de préparation d'un mononitrate de glycéryle ou d'un mélange de mononitrates de glycéryle, caractérisé en ce qu'il comprend une étape de bioconversion de la nitroglycérine en mononitrate(s) de glycéryle par un microorganisme choisi dans le groupe constitué par les levures, les fungi et les protozoaires, de préférence par bioconversion à l'aide d'un fungus.

Dans le procédé selon l'invention, il est possible d'utiliser des microorganismes produisant sélectivement le mononitrate-1 de glycéryle (MNG-1) ou bien le mononitrate-2 de glycéryle (MNG-2) ou bien des microorganismes permettant d'obtenir des mélanges de MMG-1 et MNG-2.

Parmi les nombreux microorganismes utilisables dans le cadre de la présente invention, il faut citer plus particulièrement les fungi choisis parmi les genres Cunninghamella, Geotrichum, Sporotrichum, Phanerochaete, Beauveria et Mucor. De préférence, on utilisera les espèces Cunninghamella elegans, Geotrichum candidum, Sporotrichum paranense, Geotrichum exile, Phanerochaete chysosporium, Beauveria tenella,

Beauveria bassiana et Mucor plombeus.

Parmi les protozaires, les représentants du genre Tetrahymena donnent de bons résultats, par exemple, le Tetrahymena thermophila.

Parmi les levures, le représentants des genres Rhodotorula et Hansenula sont intéressants, notamment Rhodotorula glutinis et Hansenula anomala donnent de bons résultats.

Dans la mise en oeuvre de ces microorganismes,on constate, au cours de l'étape de bioconversion de la nitroglycérine, et à la différence de ce qui est rapporté dans la technique antérieure, une disparition totale de la nitroglycérine, l'apparition puis la disparition des dinitrates de glycéryle, et l'apparition puis le maintien à une concentration constante de MNG-1 et/ou de MNG-2.

Les microorganismes peuvent être mis en oeuvre sous forme libre, sous forme liée par adsorption sur un support ou sous forme encapsulée dans un polymère naturel ou synthétique (alginate ou carraghénate, par exemple selon des techniques connues) ; dans certains cas, il est possible d'utiliser des extraits acellulaires.

La bioconversion de la nitroglycérine en mononitrate(s) de glycéryle est effectuée soit dans une solution minérale ou organique contenant le microorganisme et la nitroglycérine, soit de préférence dans un milieu de culture du microorganisme dans lequel on ajoute la nitroglycérine de préférence après croissance.

Bien qu'il soit possible d'envisager un grand nombre de milieux de culture qui soient fonction des microorganismes mis en oeuvre, le milieu contenant :

- un extrait de levure,
- un extrait de malt,
- et un sucre tel que le glucose, est particulièrement approprié.

Ainsi, en milieu liquide, il est possible d'utiliser une composition contenant :

| | |
|---|---|
| – un extrait de levure | 4-20 g/l |
| – un extrait de malt | 5-15 g/l |
| – glucose | 2-50 g/l |

Un milieu de culture contenant à titre d'extrait de levure le Difco Yeast Extract® commercialisé par la Société Difco donne de bons résulstats.

Selon un mode avantageux de la mise en oeuvre du procédé selon l'invention, la nitroglycérine est ajoutée en une seule fois au milieu de culture, à raison de 2 à 5 millimoles par litre, de préférence 3 à 4 millimoles par litre.

La nitroglycérine peut aussi être ajoutée en plusieur fois au milieu de culture. Dans ce cas, la quantité cumulée de TNG mis en oeuvre peut atteindre 12 millimoles par litre, soit environ 2,7 g de nitroglycérine par litre.

Les conditions de culture doivent être adaptées à chaque microorganisme.

La fourchette de température la plus favorable à la bioconversion est comprise entre 20 et 50°C.

Toutefois, certains fungi dits "thermophiles" peuvent être efficaces à des températures plus élevées. Dans ce cas, la durée de bioconversion est considérablement réduite, ce qui est intéressant sur le plan économique.

En général, la fourchette de pH utilisable est assez large (3-8), mais dans certains cas, il est utile et même indispensable d'ajuster plus spécifiquement le pH en fonction de la souche utilisée pour réaliser la bioconversion.

Les conditions d'agitation et d'oxygénation sont fonction de la souche mise en oeuvre et sont connues de l'homme du métier.

Comme cela a été indiqué précédemment, le procédé permet d'obtenir soit un mélange de mononitrates ou bien majoritairement l'un des mononitrates.

- obtention de MNG-1, (sélectivité de 84%) avec Cunninghamella elegans,
- obtention de MNG-1, (sélectivité de 65%) avec Geotrichum candidum,
- obtention de MNG-2, (sélectivités de 100%) avec Sporotrichum parenense,
- obtention de MNG-2 (sélectivité de 100%) avec Phanerochaete chrysoporium,
- obtention de MNG-1 (sélectivité de 60%) avec Beauveria tenella, ou avec Beauvaria bassiana,
- obtention de MNG-2 et MNG-1 en proportions égales avec Mucor plombeus ou avec Geotrichum exile.

D'autres caractéristiques et avantages du procédé selon l'invention seront illustrés par les exemples ci-après et par la figure unique qui représente la bioconversion de la nitroglycérine par Coetrichum candidum CBS 23-376 en fonction du temps.

EXEMPLE 1 Transformation de la trinitroglycérine en mononitrate-2 de glycéryle par culture de Phanerochaete chrysosporium ATCC 34541

1 – Réalisation d'une culture

– Composition du milieu de culture :

| | |
|---|---|
| . Difco Yeast Extract® | 4 g/l |
| . Malt Extract® | 10 g/l |
| . D-glucose | 4 g/l |
| . Eau distillée | qsp 1l |
| . pH ajusté à 6,8 | |

Le Difco Yeast Extract® et le Malt Extract® sont des extraits de levure et de malt commercialisés par la Société Difco.

– Stérilisation à 115°C pendant 20 minutes.

– préparation de la solution d'ensemencement contenant du Phanerochaete chrysosporium ATOC 34541 à une densité moyenne de $5.10^7$ spores/ml, en suspension dans de l'eau stérile, à laquelle on a ajouté un tensio-actif tel que le sulfonate connu sous le nom de Tween 80®.

– Ensemencement de 50 ml de milieu de culture dans un erlenmeyer de 250 ml avec 1 ml de la solution d'ensemencement.

La culture ainsi obtenue est themostatée à 24°C et agitée sur plaque rotative.

2 – Bioconversion

3 jours après l'ensemencement, on réalise stérilement les additions suivantes :

. Addition de 1 ml de solution éthanolique à 3% de nitroglycérine (110 µmol),

. Addition de 1 ml de la solution de nitroglycérine mentionnée ci-dessus 7 jours après la première.

. Addition de 1 ml de la solution de nitroglycéryne sus-mentionnée 10 jours après la seconde.

La bioconversion de la nitroglycérine au cours du temps est suivie par chromatographie haute performance en phase liquide (HPLC) de parties aliquotes du surnageant de la culture (colonne C18, méthanol à 10% comme éluant).

3 – Extraction et isolement du produit

. Centrifugation de la culture, 12 jours après la dernière addition de trinitroglycérine.

. 5 extractions successives du surnageant à l'acétate d'éthyle.

. Séchage des phases organiques au sulfate de sodium, suivi d'une évaporation sous pression réduite.

. Purification par chromotagraphie sur silice.

4 – Résultats obtenus

Les résultats, exprimés en µmol, sont obtenus par comparaison avec une solution standard contenant de la trinitroglycérine et ses dérivés d'hydrolyse.

Les appréciations des quantités ne rendent pas compte de l'évaporation du milieu qui n'est pas négligeable lorsqu'on agite le milieu de culture pendant les phases de culture et de bioconversion. Les résultats obtenus sont rassemblés dans le tableau 1.

DNG-1,3 signifie dinitrate-1,3 de glycéryle.

DNG-1,2 signifie dinitrate-1,2 de glycéryle.

TABLEAU 1 Bioconversion de la trinitroglycérine par *Phanerochaete chrysosporium* ATCC 34541

| Temps (jours) | MNG-1 µmol | MNG-2 µmol | DNG-1,3 µmol | DNG-1,2 µmol | TNG µmol |
|---|---|---|---|---|---|
| 0 | - | - | - | - | + 110 |
| 1 | 4 | 20 | 3 | 83 | < 1 |
| 2 | < 1 | 50 | 2 | 60 | < 1 |
| 3 | < 1 | 64 | < 1 | 46 | < 1 |
| 7 | < 1 | 90 | < 1 | 18 | < 1 |
| | | | | | + 110 |
| 8 | < 1 | 70 | 6 | 140 | < 1 |
| 9 | < 1 | 112 | < 1 | 106 | < 1 |
| 10 | < 1 | 146 | < 1 | 73 | < 1 |
| 17 | < 1 | 165 | 12 | 9 | < 1 |
| | | | | | + 110 |
| 18 | < 1 | 162 | 8 | 160 | < 1 |
| 22 | < 1 | 335 | < 1 | 24 | < 1 |
| 23 | < 1 | 311 | < 1 | 17 | < 1 |
| 29 | < 1 | 321 | < 1 | 10 | < 1 |

. Rendement en produit formé : 97 %
. Rendement en produit isolé : 40 %
. Sélectivité en MNG-2 :100 %

Le composé majoritaire, obtenu pur en HPLC, correspond à $C_3H_7NO_5$ d'après l'analyse élémentaire :

| | % calculé | % mesuré |
|---|---|---|
| C | 26,3 | 26,22 |
| H | 5,1 | 5,37 |
| N | 10,2 | 10,30 |

Le spectre RMN du proton obtenu en solution dans $CDCl_3$ montre que ce composé correspond au mononitrate-2 de glycéryle (l'addition de $D_2O$ provoque la disparition des 2 pics attribués aux hydrogènes des

hydroxyles 1 et 3).

EXEMPLE 2 Transformation de la trinitroglycérine en un mélange de mononitrate-1 de glycéryle et de mononitrate-2 de glycéryle par Geotrichum candidum CBS 23-376.

1 – Réalisation d'une culture

. Composition du milieu de culture identique à celle de l'exemple 1.
. Stérilisation du milieu à 115°C pendant 20 minutes.
. Ensemencement dans un erlenmeyer de 2 litres de 250 ml du milieu de culture par $2,5.10^8$ spores de Geotrichum candidum CBS 23-376 prélevées sur des pré-cultures en tubes d'agar.
. Agitation de cette culture à 24°C pendant 3 jours sur plaque rotative.

2 – Bioconversion

. Addition de 3,75 ml d'une solution à 4% de trinitroglycérine dans l'éthanol (660 µmol).
. Addition identique à la première, 7 jours plus tard.
La bioconversion de la nitroglycérine en fonction du temps est suivie de la même manière que dans l'exemple 1.

3 – Extraction et isolement des produits

Centrifugation de la culture 6 jours après la seconde addition de trinitroglycérine.
. Extraction des mononitrate-1 et mononitrate-2 de glycéryle du surnageant par 5 × 100 ml d'acétate d'éthyle.
. Obtention d'un résidu par séchage et évaporation des phases organiques.
. Dépôt de résidu tel quel sur gel de silice.
. Séparation des mononitrates de glycéryle par élution avec un mélange de $CH_2Cl_2/CH_3OH$ (95/5).

4 – Résultats

Les résultats sont exprimés en µmol dans les conditions décrites dans l'exemple 1, et sont rassemblés dans le tableau 2.

TABLEAU 2 Bioconversion de la trinitroglycérine par Géotrichum candidum CBS 23 -376

| Temps (jours) | MNG µmol | DNG-1,3 µmol | DNG-1,2 µmol | TNG µmol |
|---|---|---|---|---|
| 0 | - | - | - | + 660 |
| 1 | 10 | 215 | 280 | 150 |
| 2 | 200 | 265 | 190 | <1 |
| 3 | 495 | 110 | 50 | <1 |
| 4 | 540 | 90 | 25 | <1 |
| 7 | 620 | 25 | <1 | <1 |
| | | | | + 660 |
| 8 | 400 | 530 | 380 | <1 |
| 9 | 570 | 435 | 300 | <1 |
| 10 | 990 | 237 | 92 | <1 |
| 13 | 1250 | 43 | 21 | <1 |

. Rendement en mononitrates formés
(mélange MNG-1 et MNG-2) : 95%
. Rendement en mononitrates isolés
(mélange MN-1 et MNG-2) : 90%
. Sélectivité pour MNG-1 : 65%

La RMN du proton montre qu'il y a 2 fois plus de MNG-1 que de MNG-2 dans le mélange obtenu.

La bioconversion de la nitroglycérine par Géotrichum candidum CBS 23-276 en fonction du temps, est illustrée par la figure 1 dans laquelle :

——.——.—— représente la concentration en nitroglycérine.

----x---x--- représente la concentration en dinitrate 1, 3, de glycéryle.

----o----o-- représente la concentration en dinitrate 1, 2, de glycéryle.

——▲—— représente la concentration en mélange de MNG-1 et MNG-2.

EXEMPLE 3 Transformation de la nitroglycérine en mononitrate1 de glycéryle et en mononitrate-2 de glycéryle par culture de Cunninghamella elegans ATCC 9245

1 – Réalisation d'une culture

. Composition du milieu de culture identique à celle de l'exemple 1.

. Stérilisation du milieu selon les modalités décrites dans l'exemple 1.

. Ensemencement dans un erlenmeyer de 2 litres de 250 ml du milieu de culture par 1 ml d'une suspension de spores de Cunninghamella elegans ATCC 9245 issues d'une préculture sur un tube d'agar nutritif contenant un tensio-actif (Tween 80® à une concentration de 1 pour mille).
. La culture ainsi obtenue est thermostatée à 24°C et agitée sur plaque rotative.

2 – Bioconversion

3 jours après l'ensemencement, addition de 5 ml d'une solution éthalonique à 4% de nitroglycérine (724 µmol).
La bioconversion de la nitroglycérine au cours du temps est suivie par HPLC.

3 – Extraction

Selon les mêmes modalités que dans les exemples précédents.

4 – Résultats

Les résultats sont exprimés en µmol dans les conditions décrites par l'exemple 1, et sont rassemblés dans le tableau 3.

Tableau 3 Bioconversion de la trinitroglycérine par Cunninghamella elegans ATCC 9245

| Temps (jours) | MNG-1 µmol | MNG-2 µmol | DNG-1,3 µmol | DNG-1,2 µmol | TNG µmol |
|---|---|---|---|---|---|
| 0 | - | - | - | - | 724 |
| 1 | 55 | 25 | 700 | 275 | <1 |
| 3 | 140 | 30 | 490 | 190 | <1 |
| 10 | 300 | 80 | 260 | 75 | <1 |
| 14 | 355 | 65 | 205 | 65 | <1 |

- Rendement en mononitrates formés (mélange MNG-1 et MNG-2) : 58%
- Rendement en MNG-1 formé : 49%
- Sélectivité en MNG-1 : 84%

EXEMPLE 4 Transformation de la nitroglycérine en mononitrate-1 de glycéryle et en mononitrate-2 de glycéryle par culture de Beauveria bassiana ATCC 7159

1 – Réalisation d'une culture

- Composition du milieu de culture identique à celle de l'exemple 1.
- Stérilisation selon les modalités décrites dans l'exemple 1.
- Ensemencement dans un erlenmeyer de 250 ml, de 50 nl du milieu par des spores de Beauveria bassiana ATCC 7159 obtenues sur un tube d'agar nutritif incliné, reprises stérilement dans 1 à 5 ml d'eau contenant un tensio-actif (Tween 80® à une concentration de 1 pour mille).
- Culture à 24°C, sous agitation constante.

2 – Bioconversion

3 jours après l'ensemencement addition de 1 ml d'une solution éthanolique à 3% de nitroglycérine (110 µmol).

La bioconversion de la nitroglycérine en fonction du temps est suivie par analyse HPLC de parties aliquotes du surnageant de la culture.

3 – Extraction

Selon les mêmes modalités que dans les exemples précédents.

4 – Résultats

Les résultats sont exprimés en µmol dans les conditions écrites dans l'exemple 1 et sont rassemblés dans le tableau 4.

TABLEAU 4 Bioconversion de la nitroglycérine par *Beauveria bassiana* ATCC 7159

| Temps (jours) | MNG µmol | DNG-1,3 µmol | DNG-1,2 µmol | TNG µmol |
|---|---|---|---|---|
| 0 | - | - | - | 110 |
| 1 | 9 | 74 | 81 | 18 |
| 2 | 40 | 123 | 100 | <1 |
| 5 | 75 | 100 | 60 | <1 |
| 6 | 90 | 95 | 55 | <1 |

– Rendement en mononitrates formés (mélange MNG-1 et MNG-2) : 82%
– Sélectivité en MNG-1 : 60%

EXEMPLE 5 Transformation de la nitroglycérine en mononitrate-2 de glycéryle par culture de *Sporotrichum paranense* 1995 (Museum d'histoire naturelle-Paris)

1 – Réalisation d'une culture

– Composition du milieu de culture identique à celle décrite dans l'exemple 1.
– Stérilisation du milieu selon les modalités décrites dans l'exemple 1.
– Ensemencement, dans un erlenmeyer de 250 ml, de 50 ml de milieu par $2.10^7$ spores de *Sporotrichum paranense* 1995 (Museum d'histoire naturelle – Paris).
– Agitation constante de cette culture à 24°C.

2 – Bioconversion

– Addition de 1 ml d'une solution éthanolique à 4% de nitroglycérine (145 µmol), 3 jours après l'ensemencement.

3 – Extraction et isolement des produits

– Centrifugation du surnageant 21 jours après addition de la nitroglycérine.
– Saturation par le chlorure de sodium.

– Extraction à l'acétate d'éthyle.
– Séchage et évaporation de la phase organique.
– Purification du résidu obtenu sur plaque de gel de silice.

4 – Résultats

Les résultats sont exprimés en μmol dans les conditions décrites dans l'exemple 1 et sont rassemblés dans le tableau 5.

TABLEAU 5 Bioconversion de la nitroglycérine par Sporotrichum paranense 1995 (Museum d'histoire naturelle-Paris)

| Temps (jours) | MNG-1 μmol | MNG-2 μmol | DNG-1,3 μmol | DNG-1,2 μmol | TNG μmol |
|---|---|---|---|---|---|
| 0 | - | - | - | - | 145 |
| 1 | 0 | 5 | 10 | 40 | 20 |
| 8 | <1 | 70 | 20 | 120 | <1 |
| 15 | <1 | 90 | 6 | 45 | <1 |
| 21 | <1 | 105 | 7 | 30 | <1 |

– Rendement en MNG-2 formé : 73%
– Sélectivité en MNG-2 : 100%
– Rendement en MNG-2 isolé : 40%

EXEMPLE 6 Transformation de la nitroglycérine en mononitrate-1 de glycèryle et en mononitrate-2 de glycéryle par culture de Mucor plombeus CBS 24658

Dans les mêmes conditions que celles décrites dans l'exemple 1, on réalise une culture par ensemencement de 50 ml du milieu de culture avec les spores de Mucor plombeus CBS 24658, issues d'une pré-culture en tube d'agar nutritif incliné.

Après 3 jours sous agitation à 24°C, on ajoute au milieu de culture 1 ml d'une solution éthanolique à 4% de nitroglycérine. Après 10 jours, le rendement en mélange de MNG-1 et MNG-2 formés est de 50%, avec une proportion égale de MNG-1 et MNG-2.

EXEMPLE 7 Transformation de la nitroglicérine en mononitrate-1 de glycéryle et en mononitrate-2 de glycéryle par culture du protozoaire Tetrahymena thermophila B4-1868

1 – Réalisation d'une culture

– Composition du milieu de culture
. bactopeptone 20 g/l
. D-glucose 5 g/l
. extrait de levure 1 g/l
. $MgSO_4$, $6H_2O$ 0,25 g/l
. $KH_2PO_4$ 0,5 g/l
. EDTA ferrique 30 mg/l
– Stérilisation à 115°C pendant 20 minutes.
– Ensemencement dans un erlenmeyer de 250 ml, de 50 ml de milieu par 1 à 2 ml d'une suspension aqueuse de Tetrahymena thermophila B4-1868.

La culture ainsi obtenue est thermostatée à 28°C et maintenue sous agitation douce et linéaire (de l'ordre de 100 va et vient par minute).

Les cellules sont comptées au microscope.

2 – Bioconversion

– Addition de 33 µmol de nitroglycérine en solution à 4% dans l'éthanol, quand la culture atteint une densité de $35.10^5$ cellules dans 50 ml de milieu.

– Agitation et température maintenues dans les mêmes conditions que pour la culture.

L'évolution de la bioconversion en fonction du temps est suivie par HPLC de parties aliquotes du surnageant.

3 – Extraction

Selon les mêmes modalités que dans les exemples précédents.

4 – Résultats

Les résultats sont donnés en µmol, dans les conditions décrites dans l'exemple 1 et sont rassemblés dans le tableau 6.

TABLEAU 6 Bioconversion de la nitroglycérine par Tétrahymena thermophila B4-1868

| Temps (jours) | nombre cellules | MNG-2 µmol | MNG-1 µmol | DNG-1,3 µmol | DNG-1,2 µmol | TNG µmol |
|---|---|---|---|---|---|---|
| 0 | $35.10^5$ | - | - | - | - | 33 |
| 1 | $30.10^6$ | 9,5 | 8,6 | 7 | 10 | <1 |
| 2 | $5.10^7$ | pas de résultat | 5,5 | 9 | 20 | <1 |
| 3 | $10^7$ | 5,7 | 5,3 | 7,7 | 14 | <1 |
| 6 | $10^6$ | 7 | 6,6 | 7,4 | 12 | <1 |

L'hydrolyse de la nitroglycérine par Tetrahymena thermophila B4-1868 est rapide et efficace ; elle conduit à un mélange à peu près équimoléculaire de mononitrates de glycéryle. Toutefois, le substrat et les produits obtenus sont fortement dilués pour préserver la survie du protozoaire. Si on administre une dose de 70 µmol de nitroglycérine (en solution éthanolique 4%) dans 50 ml, c'est-à-dire 1,4 millimolaire, les cellules meurent dans la première journée tout en hydrolisant complètement la nitroglycérine.

EXEMPLE 8 Transformation de la nitroglycérine en mononitrate1 de glycéryle et en mononitrate-2 de glycéryle par culture de la levure Rhodotorula glutinis ATCC 15125

1 – Réalisation d'une culture

– Composition du milieu identique à celle décrite dans l'exemple 1.

– Stérilisation du milieu à 115°C pendant 20 minutes.

– Ensemencement dans un erlenmeyer de 250 ml, de 50 ml de milieu avec une petite quantité de cellules de Rhodotorula glutinis ATCC 15125.

La culture ainsi obtenue est thermostatée à 24°C et maintenue sous agitation constante (plaque rotative, 180 tours/minute).

2 – Bioconversion

– Addition de 1 ml d'une solution de nitroglycérine à 3% dans l'éthanol (107 µmol), 3 jours après l'ensemencement.
– L'incubation se déroule dans les mêmes conditions que pour la culture.
L'évolution de la bioconversion au cours du temps est suivie par HPLC de parties aliquotes traitées préalablement sur silice.

3 – Extraction

Selon les modalités décrites précédemment.

4 – Résultats

Les résultats sont exprimés en µmol selon les mêmes conditions que celles décrites dans l'exemple 1, et sont résumés dans le tableau 7.

TABLEAU 7 Bioconversion de nitroglycérine par Rhodotorula glutinis ATCC 15125

| Temps (jours) | MNG µmol | DNG-1,3 µmol | DNG-1,2 µmol | TNG µmol |
|---|---|---|---|---|
| 0 | - | - | - | 107 |
| 1 | <1 | 22 | 6 | 72 |
| 2 | <1 | 39 | 27 | 62 |
| 3 | <1 | 43 | 38 | 47 |
| 4 | 8 | 30 | 28 | 40 |

CONCLUSION : La levure est capable d'hydrolyser la nitroglycérine en mononitrates de glycéryle. Cependant, dans ce cas particulier, les réactions sont lentes.

## Revendications

1. Procédé de préparation d'un mononitrate de glycéryle ou d'un mélange de mononitrates de glycéryle caractérisé en ce qu'il comprend une étape de bioconversion de la nitroglycéryne en mononitrate(s) de glycéryle par un microorganisme choisi dans le groupe constitué par les levures, les fungi et les protozoaires, de préférence une étape de bioconversion par les fungi.

2. Procédé selon la revendication 1, caractérisé en ce que les fungi sont choisis parmi les genres Cunninghamella, Geotrichum, Sporotrichum, Phanerochaete, Beauveria et Mucor, et de préférence parmi les espèces Cunninghamella elegans, Geotrichum candidum, Sporotrichum paranense, Geotrichum exile, Phanerochaete chrysosporium, Beauveria tenella, Beauveria bassiana et Mucor plombeus.

3. Procédé selon la revendication 1, caractérisé en ce que les protozoaires appartiennent au genre Tetrahymena, et que les levures appartiennent au genre Rhodotorula.

4. Procédé selon l'une quelconque des revendications 1 à 3, caractérisé en ce que les microorganismes sont mis en oeuvre sous forme libre, ou bien sous forme liée, ou bien sous forme encapsulée, par exemple, dans un polymère naturel ou synthétique.

5. Procédé selon l'une quelconque des revendications 1 à 4, caractérisé en ce que la bioconversion est effectuée dans un milieu de culture du microorganisme comportant de la nitroglycérine.

6. Procédé selon la revendication 5, caractérisé en ce que la nitroglycérine est ajoutée au milieu de culture

en une seule fois, à raison de 2 à 5 millimoles par litre, de préférence 3 à 4 millimoles par litre de milieu.

7. Procédé selon l'une quelconque des revendications 5 et 6, caractérisé en ce que le milieu de culture dans lequel s'effectue la bioconversion contient :

– extrait de levure 4 à 20 g/l
– extrait de malt 5 à 15 g/l
– glucose stérilisé 2 à 50 g/l

8. Procédé selon l'une quelconque des revendications 5 à 7, caractérisé en ce que la bioconversion a lieu à une température comprise entre 20° et 50°C, et que le pH du milieu de culture est compris entre 3 et 8.

9. Procédé selon l'une quelconque des revendications 1, 2, 4, 5, 6, 7 ou 8, caracterisé en ce que l'on prépare majoritairement le mononitrate-2 de glycéryle par mise en oeuvre de Sporotrichum paranense ou de Phanerochaete chrysosporium.

10. Procédé selon l'une quelconque des revendications 1, 2, 4, 5, 6, 7 ou 8, caractérisé en ce que l'on préparera majoritairement le mononitrate-1 de glycéryle par mise en oeuvre de Cunninghamella elegans, ou de Beauveria tenella, ou de Geotrichum candidum.

## Ansprüche

1. Verfahren zur Herstellung eines Glycerinmononitrats oder eines Gemischs der Glycerinmononitrate, dadurch gekennzeichnet, daß es einen Schritt der Biokonversion von Glycerintrinitrat zu Glycerinmononitrat(en) durch einen unter Hefen, Pilzen und Protozoen ausgewählten Mikroorganismus, vorzugsweise einen Schritt der Biokonversion durch Pilze, umfaßt.

2. Verfahren nach Anspruch 1, dadurch gekennzeichnet, daß die Pilze unter den Gattungen Cunninghamella, Geotrichum, Sporotrichum, Phanerochaeta, Beauveria und Mucor und vorzugsweise unter den Arten Cunninghamella elegans, Geotrichum candidum, Sporotrichum paranense, Geotrichum exile, Phanerochaeta chrysosporium, Beauveria tenella, Beauveria bassiana und Mucor plombeus ausgewählt werden.

3. Verfahren nach Anspruch 1, dadurch gekennzeichnet, daß die Protozoen zur Gattung Tetrahymena und die Hefen zur Gattung Rhodotorula gehören.

4. Verfahren nach einem der Ansprüche 1 bis,3, dadurch gekennzeichnet, daß die Mikroorganismen in freier, in gebundener oder in eingekapselter Form, beispielsweise eingekapselt in einem natürlichen oder synthetischen Polymer, eingesetzt werden.

5. Verfahren nach einem der Ansprüche 1 bis 4, dadurch gekennzeichnet, daß die Biokonversion in einem Kulturmedium des Mikroorganismus durchgeführt wird, das Glycerintrinitrat enthält.

6. Verfahren nach Anspruch 5, dadurch gekennzeichnet, daß das Glycerintrinitrat dem Kulturmedium auf einmal in einer Menge von 2 bis 5 mmol pro Liter und vorzugsweise von 3 bis 4 mmol pro Liter Medium zugegeben wird.

7. Verfahren nach Anspruch 5 oder 6, dadurch gekennzeichnet, daß das Kulturmedium, in dem die Biokonversion durchgeführt wird, enthält:

Hefeextrakt 4 bis 20 g/l
Malzextrakt 5 bis 15 g/l
Sterilisierte Glucose 2 bis 50 g/l

8. Verfahren nach einem der Ansprüche 5 bis 7, dadurch gekennzeichnet, daß die Biokonversion bei einer Temperatur von 20 bis 50°C und einem pH-Wert des Kulturmediums von 3 bis 8 durchgeführt wird.

9. Verfahren nach einem der Ansprüche 1, 2, 4, 5, 6, 7 oder 8, dadurch gekennzeichnet, daß durch Verwendung von Sporotrichum paranense oder von Phanerochaeta chrysosporium überwiegend Glycerin-2-mononitrat hergestellt wird.

10. Verfahren nach einem der Ansprüche 1, 2, 4, 5, 6, 7 oder 8, daß durch gekennzeichnet, dadurch Verwendung von Cunninghamella elegans, Beauveria tenella oder von Geotrichum candidum überwiegend Glycerin-1-mononitrat hergestellt wird.

## Claims

1. Process for preparing a glyceryl mononitrate or a mixture of glyceryl mononitrates, characterised in that it comprises a step of bioconversion of nitroglycerin to glyceryl mononitrate(s) by a microorganism selected from the group consisting of yeasts, fungi and protozoa, preferably a step of bioconversion by fungi.

2. Process according to Claim 1, characterised in that the fungi are selected from the genera Cunninghamella, Geotrichum, Sporotrichum, Phanerochaete, Beauveria and Mucor, and preferably from the species

Cunninghamella elegans, Geotrichum candidum, Sporotrichum paranense, Geotrichum exile, Phanerochaete chrysosporium, Beauveria tenella, Beauveria bassiana and Mucor plombeus.

3. Process according to Claim 1, characterised in that the protozoa belong to the genus Tetrahymena, and in that the yeasts belong to the genus Rhodotorula.

4. Process according to any one of Claims 1 to 3, characterised in that the microorganisms are employed in free form, or alternatively in bound form, or alternatively in encapsulated form, for example, in a natural or synthetic polymer.

5. Process according to any one of Claims 1 to 4, characterised in that the bioconversion is performed in a medium for culture of the microorganism containing nitroglycerin.

6. Process according to Claim 5, characterised in that the nitroglycerin is added to the culture medium in a single portion, in a proportion of 2 to 5 millimoles per litre and preferably 3 to 4 millimoles per litre of medium.

7. Process according to either of Claims 5 and 6, characterised in that the culture medium in which the bioconversion is performed contains :

| | |
|---|---|
| – yeast extract | 4 to 20 g/l |
| – malt extract | 5 to 15 g/l |
| – sterilised glucose | 2 to 50 g/l |

8. Process according to any one of Claims 5 to 7, characterised in that the bioconversion takes place at a temperature of between 20° and 50°C, and in that the pH of the culture medium is between 3 and 8.

9. Process according to any one of Claims 1, 2, 4, 5, 6, 7 or 8, characterised in that glyceryl 2-mononitrate is prepared as the preponderant product by employing Sporotrichum paranense or Phanerochaete chrysosporium.

10. Process according to any one of Claims 1, 2, 4, 5, 6, 7 or 8, characterised in that glyceryl 1-mononitrate is prepared as the preponderant product by employing Cunninghamella elegans, or Beauveria tenella or Geotrichum candidum.

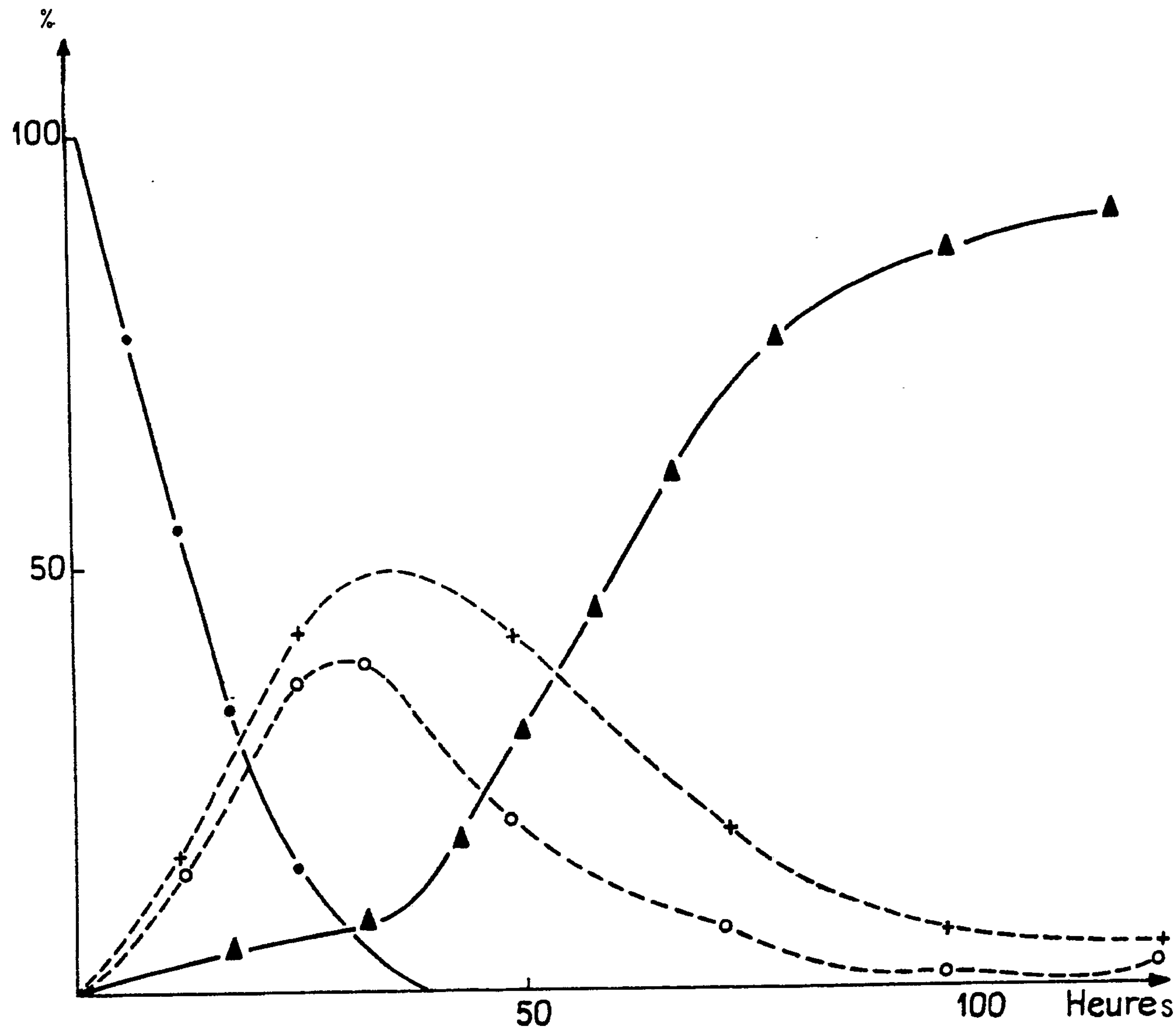
%
100
50
50
100
Heures